# EUROPEAN PATENT SPECIFICATION

(11) **EP 1 270 205 B1**
(45) Date of publication and mention of the grant of the patent: **31.03.2004**
(21) Application number: 02013288.2
(22) Date of filing: 18.06.2002
(51) Int. Cl.: A61L 15/44, A61F 13/02, A61L 15/26, A61L 15/58, B32B 27/12, B32B 27/36

(54) **Adhesive sheet and adhesive preparation, and production methods thereof**
Klebefolie und Klebstoffzubereitung und Verfahren zur Herstellung
Feuille adhésive et préparation adhésive et procédé de fabrication

(30) Priority: 19.06.2001 JP 2001185581
(43) Date of publication of application: 02.01.2003
(73) Proprietor: NITTO DENKO CORPORATION, Ibaraki-shi, Osaka 567-8680 (JP)
(72) Inventor: Hori, Mitsuhiko, Ibaraki-shi, Osaka 567-8680 (JP); Ninomiya, Kazuhisa, Ibaraki-shi, Osaka 567-8680 (JP); Matsuoka, Kensuke, Ibaraki-shi, Osaka 567-8680 (JP); Yamamoto, Keiji, Ibaraki-shi, Osaka 567-8680 (JP); Minomi, Kenjiro, Ibaraki-shi, Osaka 567-8680 (JP); Nakano, Yoshihisa, Ibaraki-shi, Osaka 567-8680 (JP)
(74) Representative: von Kreisler, Alek, Dipl.-Chem.

(56) References cited:
- EP-A- 0 247 539
- EP-A- 0 569 862
- PATENT ABSTRACTS OF JAPAN vol. 1996, no. 08, 30 August 1996 (1996-08-30) & JP 08 092074 A (NITTO DENKO CORP), 9 April 1996 (1996-04-09)

## Description

### FIELD OF THE INVENTION

The present invention relates to an adhesive sheet and an adhesive preparation that are adhered to a skin surface for protection of a wound and the like and for continuous administration of a drug into a body from the skin, and to a production methods thereof.

### BACKGROUND OF THE INVENTION

Adhesive sheets containing a large amount of an organic liquid component in an adhesive layer have been developed in recent years. Such adhesive sheets have many advantages in that they show superior adhesion to the skin, can be applied again after peeling off, are less associated with physical skin irritation caused by peeling of an adhesive sheet, and so on.

In percutaneous absorption type adhesive preparations containing various drugs in an adhesive layer of an adhesive sheet, one containing, in an adhesive layer, a large amount of an organic liquid component (e.g., percutaneous absorption promoter etc.) has been developed to increase skin permeability of the drug, in addition to the aforementioned beneficial characteristics of superior adhesiveness, re-applicability and lowered physical irritation upon peeling.

As mentioned above, addition of an organic liquid component to an adhesive layer of an adhesive sheet or an adhesive preparation is extremely useful. However, when an organic liquid component is added in a large amount, the adhesive is plasticized to reduce its cohesiveness, which then causes problems in that the adhesive partially remains on the skin upon peeling of the adhesive sheet and adhesive preparation (i.e., adhesive residue), and a part of an adhesive or organic liquid component leaks out from the edge of the adhesive layer during preservation of an adhesive sheet and an adhesive preparation in a package and adheres to the inside of the package, thereby preventing the adhesive sheet and the adhesive preparation from being taken out easily, and the like.

To prevent such conventional decrease in the cohesiveness of an adhesive and to solve the aforementioned problems, various crosslinking agents such as isocyanate, metal salt, epoxy and the like have been added to the adhesive layer. In the conventional adhesive sheets and adhesive preparations, however, because the crosslinking agent is directly added to an adhesive coating solution, crosslinking proceeds in the coating solution during the period of from addition of the crosslinking agent to the termination of the application, and the viscosity increases to make the coating difficult (shortened pot life). In addition, isocyanate crosslinking agents react with a basic drug (e.g., tulobuterol, biperidene and the like) when the drug is contained in an adhesive layer. It is therefore inapplicable because it inhibits crosslinking of the adhesive layer.
JP-A-08-092074 discloses a plaster which is produced by forming an undercoating layer composed of an ethleneimine-modified acrylic polymer or an polyethyleneimine on one surface of a substrate and forming a tacky adhesive layer on the undercoating layer. An alkyl acrylate polymer is preferred as the tacky adhesive layer. A plaster for transcutaneous absorption therapy is disclosed which includes a drug in the tacky adhesive layer. The drug may be a crystalline or amorphous substance.

EP-A-0569862 discloses a medical adhesive sheet comprising a support comprising a laminate of a polyester film having a thickness of from 0.5 to µm and a polyester nonwoven fabric having a basis weight of from 5 to 20 g/m², and a pressure-sensitive adhesive layer laminated on the surface of the nonwoven fabric. The pressure-sensitive adhesive is an acrylic pressure-sensitive adhesive made from (meth)acrylic acid alkyl ester. Furthermore, a medical preparation is disclosed which comprises said adhesive sheet and in which said adhesive layer contains a drug.

### SUMMARY OF THE INVENTION

It is therefore an object of the present invention to provide a novel adhesive sheet and an adhesive preparation, that have solved the above-mentioned problems, and production methods thereof.

According to the present invention, an adhesive sheet comprising a substrate comprising a synthetic resin film and a nonwoven fabric laminated on one side of the film, and an adhesive layer formed on the nonwoven fabric, wherein the substrate comprises polyethyleneimine at least on a surface of the nonwoven fabric is provided, which solves the above-mentioned problems.

Accordingly, the present invention provides:
[1] An adhesive sheet comprising a substrate comprising a synthetic resin film and a nonwoven fabric laminated on one side of the film, and an adhesive layer formed on the nonwoven fabric, wherein the nonwoven fabric comprises polyethyleneimine at least on a surface of the nonwoven fabric;
[2] the adhesive sheet according to the above-mentioned [1], wherein the polyethyleneimine is contained in a proportion of 0.01-10 g/m² and at least on the surface of the nonwoven fabric;
[3] the adhesive sheet according to the above-mentioned [1] or [2], wherein the polyethyleneimine has a number average molecular weight of 300-100,000;
[4] the adhesive sheet according to the above-mentioned [1], wherein the adhesive layer comprises an adhesive containing a carboxyl group;
[5] the adhesive sheet according to the above-mentioned [4], wherein the adhesive comprises a polymer containing the following (a) and (b):
   (a) at least one C₄₋₁₃ alkyl (meth)acrylate monomer unit; and
   (b) at least one ethylenically unsaturated monomer unit containing a carboxyl group;
[6] the adhesive sheet according to any of the above-mentioned [1] to [5], wherein the synthetic resin film is made from a polyester and the nonwoven fabric is made from a polyester;
[7] an adhesive preparation comprising a drug in an adhesive layer of the adhesive according to any of the above-mentioned [1] to [6];
[8] a production method of the adhesive sheet of the above-mentioned claim 1, which comprises forming an adhesive layer on a nonwoven fabric containing polyethyleneimine; and
[9] a production method of the adhesive preparation of claim 7, which comprises forming an adhesive layer containing a drug on a nonwoven fabric containing polyethyleneimine.

### DETAILED DESCRIPTION OF THE INVENTION

The present invention is explained in detail in the following.

The adhesive sheet of the present invention comprises a substrate comprising a synthetic resin film and a nonwoven fabric laminated on one side of the film, and an adhesive layer formed on the nonwoven fabric, and is characterized in that the nonwoven fabric comprises polyethyleneimine at least on a surface of the nonwoven fabric.

### 1. Substrate

The substrate to be used for the adhesive sheet of the present invention has a structure wherein a nonwoven fabric is laminated on one side of a synthetic resin film.

The synthetic resin film is produced from a polymer material generally used in the field of adhesive sheets. The polymer material to be used for the synthetic resin film is exemplified by polyester, polypropylene, polyethylene, ethylene/vinyl acetate copolymer and the like, preferably polyester and polyethylene. Of these, polyester is preferable, because, when a drug is contained in the adhesive preparation, the drug does not easily move to the substrate.

The synthetic resin film has a thickness of generally 1-1000 µm, preferably 2-100 µm. In view of the flexibility and operability, it is more preferably 5-50 µm.

The nonwoven fabric is produced from a material generally used in the field of adhesive sheets. Examples of the material include polyester, polyethylene, polypropylene and the like, preferably polyester and polypropylene. Polyester is most preferable because the drug in the adhesive layer is not adsorbed easily. The nonwoven fabric has a basic weight of generally 1-100 g/m², preferably 6-50 g/m², wherein 6-30 g/m² is more preferable because flexibility and adhesion to the skin surface during adhesion are fine.

When forming an adhesive layer on the nonwoven fabric, the above-mentioned synthetic resin film and the above-mentioned nonwoven fabric need only be adhered to each other. The method for adhering a synthetic resin film to a nonwoven fabric is not particularly limited. For example, a method of adhering with a pressure sensitive adhesive or adhesive, a method of adhering by heat sealing and the like are used. In view of superior strength after lamination, a method of adhering with an adhesive is preferable. The adhesion strength of each layer is generally 0.5-1000 g/12 mm, preferably 1.0-200 g/12 mm. As used herein, by the unit "g/12 mm" of the adhesion strength is meant a unit obtained by converting the adhesion strength of the substrate having various widths to a value per width 12 mm, and does not intend to limit the width of the substrate to 12 mm alone.

### 2. Polyethylenimine

The polyethyleneimine used in the present invention is a polymer compound having a repeating unit represented by the formula -CH₂CH₂NH-. It is postulated that, when polyethyleneimine is applied to a nonwoven fabric, the imino group contained in polyethyleneimine forms an ionic bond or amide bond with various functional groups of the monomer unit forming the adhesive layer to be mentioned below, which increases cohesiveness of the adhesive layer. In the present invention, polyethyleneimine is applied to at least a surface of the nonwoven fabric, rather than incorporating the same in an adhesive layer. This has an effect that conventional problems of short pot life of an adhesive and difficult formation of an adhesive layer can be obliterated.

In the present invention, polyethyleneimine generally having a number average molecular weight of 300-100,000, preferably 10,000-80,000, is used. The number average molecular weight of less than 300 is not preferable, because the reactivity between the imino group in polyethyleneimine and a functional group of a monomer unit forming an adhesive layer becomes high, and the cohesiveness of the adhesive layer becomes too high. On the other hand, a number average molecular weight of more than 100,000 is not preferable, because the reactivity between the imino group and the functional group becomes poor, and the cohesiveness of the adhesive layer becomes insufficient. Examples of the polyethyleneimine to be used in the present invention include EPOMIN® SP series (e.g., SP-003, SP-006, SP-012, SP-018, SP-200, SP-103, SP-110 etc.), EPOMIN P-1000 (all manufactured by Nippon Shokubai Co., Ltd.), and the like. These may be used alone or in combination of more than two.

The above-mentioned polyethyleneimine is applied at least to the surface of a nonwoven fabric generally at 0.01-10 g/m², preferably 0.1-5 g/m² (solid content). When the amount of polyethyleneimine to be applied is less than 0.01 g/m², the cohesiveness of the adhesive layer cannot be increased sufficiently, and an adhesive residue of the obtained adhesive sheet and adhesive preparation may be produced, or they may not be easily taken out from a package. In contrast, when the amount of polyethyleneimine to be applied exceeds 10 g/m², polyethyleneimine may easily bleed out from a substrate, and handling property of the adhesive sheet and adhesive preparation becomes poor.

The above-mentioned polyethyleneimine can be applied to a nonwoven fabric by various methods. The above-mentioned polyethyleneimine need only be applied to the surface of a nonwoven fabric of an adhesive sheet and an adhesive preparation. For example, polyethyleneimine diluted with a solvent may be applied to the surface of a nonwoven fabric and then dried, or a nonwoven fabric may be immersed in a solution containing polyethyleneimine and dried. It is preferably applied only to the surface of a nonwoven fabric, because the amount of polyethyleneimine can be economically decreased.

### 3. Adhesive layer

As the adhesive layer of the adhesive sheet or the adhesive preparation of the present invention, any adhesive containing any material can be used. Particularly, an adhesive having a carboxyl group is preferably used. When such adhesive is used for an adhesive layer, the carboxyl group reacts with the imino group of polyethyleneimine on the surface of the nonwoven fabric to form crosslinking by ionic crosslinking and/or amide bond, as a result of which the cohesiveness of the adhesive layer can be markedly increased.

Examples of the adhesive containing the above-mentioned carboxyl group include those containing alkyl (meth)acrylate polymer having a carboxyl group, silicone polymer having a carboxyl group, synthetic rubber having a carboxyl group and the like. In the present invention, an adhesive containing alkyl (meth)acrylate polymer having a carboxyl group is preferable because adhesion properties can be controlled easily.

As the above-mentioned alkyl (meth)acrylate polymer having a carboxyl group, a unit derived from (meth)acrylate monomer wherein at least one alkyl moiety is C₄₋₁₃ alkyl (hereinafter to be referred to as C₄₋₁₃ alkyl (meth)acrylate monomer unit) and a polymer containing an ethylenically unsaturated monomer unit containing at least one carboxyl group are preferable. As the alkyl moiety of the C₄₋₁₃ alkyl (meth)acrylate monomer, straight chain or branched chain C₄₋₁₃ alkyl (e.g., butyl, isobutyl, t-butyl, pentyl, isopentyl, neopentyl, hexyl, isohexyl, 2-ethylhexyl, heptyl, octyl, nonyl, decyl, undecyl, dodecyl, tridecyl, and the like) is exemplified. As the ethylenically unsaturated monomer unit having a carboxyl group, for example, a monomer unit derived from (meth)acrylic acid, itaconic acid, maleic acid, maleic anhydride and the like is mentioned.

In addition, the above-mentioned alkyl (meth)acrylate having a carboxyl group may contain other comonomer units, as long as the property as an adhesive is not impaired. Such other comonomer units are derived from the following. Alkyl (meth)acrylate wherein the alkyl moiety is C₁₋₃ alkyl (hereinafter to be referred to as C₁₋₃ alkyl (meth)acrylate monomer, e.g., (meth)acrylate monomer having methyl group, ethyl group or propyl group etc.); ethylenically unsaturated monomer having other various substituents (e.g., vinyl monomer such as (meth)acrylonitrile, vinyl acetate, vinyl propionate, N-vinyl-2-pyrrolidone, methyl vinyl pyrrolidone, vinylpyridine, vinylpiperazine, vinylpiperidone, vinylpyrimidine, vinylpyrazine, vinylpyrrole, vinylimidazole, vinylcaprolactam, vinyloxazole, vinylmorpholine, and the like) and the like.

Preferable alkyl (meth)acrylate containing a carboxyl group used in the present invention includes, for example, 2-ethylhexyl acrylate/acrylic acid copolymer, 2-ethylhexyl acrylate/vinylpyrrolidone/acrylic acid copolymer, isononyl acrylate/methoxyethyl acrylate/acrylic acid copolymer, 2-ethylhexyl acrylate/vinyl acetate/methacrylic acid copolymer, 2-ethylhexyl acrylate/methacrylic acid copolymer, 2-ethylhexyl acrylate/isobutyl acrylate/acrylic acid copolymer, and the like.

The above-mentioned C₄₋₁₃ alkyl (meth)acrylate monomer, ethylenically unsaturated monomer containing a carboxyl group and other comonomers are copolymerized according to a polymerization method known in the art. The weight ratio of the monomer units in the copolymerization is generally [C₄₋₁₃ alkyl (meth)acrylate monomer unit:ethylenically unsaturated monomer unit containing carboxyl group] = [99:1]-[50:50], preferably [97:3]-[70:30]. When other comonomer unit is contained in the copolymer, [C₄₋₁₃ alkyl (meth)acrylate monomer unit:ethylenically unsaturated monomer unit containing carboxyl group:other comonomer unit] is generally [96:1:3]-[5:50:45], preferably [90:3:7]-[50:30:20].

The thickness of the above-mentioned adhesive layer is determined in view of the thickness of the nonwoven fabric. The thickness of the adhesive layer is generally 1-5 folds, preferably 1.5-3 folds, of the thickness of the nonwoven fabric. When the thickness of the adhesive layer is smaller than that of the nonwoven fabric, the adhesive layer sinks in the nonwoven fabric and sufficient skin adhesion is not afforded. In contrast, when the thickness of the adhesive layer exceeds 5 times the thickness of the nonwoven fabric, polyethyleneimine applied to the nonwoven fabric and the adhesive may not be partially brought into sufficient contact, at which part the cohesiveness of the adhesive may become insufficient. The thickness of the adhesive layer is not particularly limited as long as the above-mentioned relationship with the thickness of the nonwoven fabric is satisfied, and is generally 10-200 µm, preferably 10-120 µm, more preferably 20-100 µm.

By adding various drugs into the adhesive layer, the above-mentioned adhesive can be formed into an adhesive preparation. The drug is not particularly limited as long as it permits percutaneous absorption. Examples thereof include general anesthetic, hypnotic drug, abirritant, antiepileptic drug, antipyretics, antivertiginous drug, psychotherapeutic drug, topical anesthetic, skeletal muscle relaxant, autonomic drug, anticonvulsant, antiparkinsonism drug, antihistamic agent, heart stimulant, antiarrhythmic agent, diuretic drug, antihypertensive agent, angiotonic, coronary vasodilator, peripheral vasodilator, drug for arteriosclerosis, circulatory drug, respiratory stimulant, antitussive and expectorant drug, hormone drug, external drug for purulent disease, analgesics, antipruritics, astringents and antiinflammatory drug, drug for parasitic skin disease, haemostatica, antarthritic, antidiabetic drug, anticancer drug, antibiotics, chemotherapeutic drug, antasthmatic, narcotic drug and the like.

The proportion of the drug in the adhesive layer in the above-mentioned adhesive preparation can be determined as appropriate in consideration of the drug releaseability of the adhesive, the kind of the drug, a pharmacological effect and the like. Preferably, the drug is added to the adhesive layer in a proportion of 0.1-70 wt%, more preferably 1-60 wt%.

The adhesive layer of the above-mentioned adhesive sheet and adhesive preparation may contain various additives as necessary. Examples of the additive include those that change solubility and diffusion properties of the drug in the adhesive layer, such as fats and oils (e.g., olive oil, squalene, lanolin etc.); plasticizers (e.g., diisopropyl adipate, phthalate, diethyl sebacate etc.); various emulsifiers; hydrocarbons (e.g., liquid paraffin etc.); and the like, additives that change skin permeability of the drug, such as glycols (e.g., diethylene glycol, propylene glycol, polyethylene glycol etc.); urea derivatives (e.g., urea, allantoin etc.); polar solvents (e.g., dimethyldecyl phosphoxide, methyloctyl sulfoxide, dimethyllaurylamide, dodecylpyrrolidone, isosorbitol, dimethylacetamide, dimethylsulfoxide, dimethylformamide etc.); higher fatty acid esters (e.g., isopropyl myristate, octyl palmitate etc.); glycerine esters (e.g., glycerine monoester such as ethoxylated stearyl alcohol, oleic monoglyceride, capric monoglyceride, lauryl monoglycerides etc.), glycerine diester, glycerine triester, mixtures thereof, and the like); and the like; and the like.

While the amount of the above-mentioned additives varies depending on the kind, the amount and the like of polyethyleneimine, adhesive, drug and the like, it is generally 0.05-2 parts by weight, preferably 0.1-1.5 parts by weight, per 1 part by weight of the adhesive.

### 4. Production of adhesive sheet and adhesive preparation

The adhesive sheet and adhesive preparation of the present invention can be produced by a method comprising directly applying an organic solvent (e.g., ethyl acetate, toluene, hexane and the like) solution (containing drug in the case of adhesive preparation) of the adhesive onto a nonwoven fabric containing polyethyleneimine, drying and adhering the same to a release liner (e.g., polyester film having a surface treated for peeling, and the like), which step is what is called direct coating. Alternatively, they may be produced by a method comprising applying an adhesive solution onto a release liner, drying to give an adhesive layer and adhering the adhesive layer to a surface of the nonwoven fabric containing polyethyleneimine, which step is what is called transfer coating. Production by direct coating is generally more preferable because direct coating provides superior contact between the adhesive layer and polyethyleneimine. When an adhesive sheet and an adhesive preparation are produced by transfer coating, a heat roll treatment and the like are preferably applied after transfer coating to make the adhesive layer permeate sufficiently into the deep part of the nonwoven fabric.

### Examples

The present invention is explained in detail by referring to examples. The examples are mere exemplifications and do not limit the present invention in any way.

### Example 1 preparation of adhesive sheet

An adhesive solution containing 60 parts by weight (solid content) of a copolymer comprising 2-ethylhexyl acrylate (95 parts by weight) and acrylic acid (5 parts by weight) and diethyl sebacate (40 parts by weight) in ethyl acetate (300 parts by weight) was prepared. This adhesive solution was applied to the surface of a nonwoven fabric of a substrate comprising a synthetic resin film (polyester film, thickness 6 µm) and a polyester nonwoven fabric [polyethyleneimine (EPOMIN P-1000, number average molecular weight 70,000) 0.5 g/m² was applied in advance, basis weight 8 g/cm², thickness 30 µm], such that the thickness after drying became 40 µm. The substrate was dried at 100°C for 5 minutes and adhered to a release liner (polyester film having a surface subjected to peel treatment) to give an adhesive sheet of the present invention.

### Example 2 preparation of adhesive sheet

An adhesive solution containing 60 parts by weight (solid content) of a copolymer comprising 2-ethylhexyl acrylate (75 parts by weight), vinyl pyrrolidone (22 parts by weight) and acrylic acid (3 parts by weight) and isopropyl myristate (40 parts by weight) in ethyl acetate (300 parts by weight) was prepared. This adhesive solution was applied to the surface of a nonwoven fabric of a substrate comprising a synthetic resin film (polyester film, thickness 6 µm) and a polyester nonwoven fabric [polyethyleneimine (EPOMIN P-1000, number average molecular weight 70,000) 0.75 g/m² was applied in advance, basis weight 8 g/cm², thickness 30 µm], such that the thickness after drying became 50 µm. The substrate was dried at 100°C for 5 minutes and adhered to a release liner (same as that used in Example 1) to give an adhesive sheet of the present invention.

### Example 3 preparation of adhesive preparation

An adhesive solution containing 50 parts by weight (solid content) of a copolymer comprising 2-ethylhexyl acrylate (75 parts by weight), vinyl pyrrolidone (22 parts by weight) and acrylic acid (3 parts by weight), isopropyl myristate (40 parts by weight) and biperidene as a drug (10 parts by weight) in ethyl acetate (300 parts by weight) was prepared. This adhesive solution was applied to the surface of a nonwoven fabric of a substrate comprising a synthetic resin film (polyester film, thickness 6 µm) and a polyester nonwoven fabric [polyethyleneimine (EPOMIN P-1000, number average molecular weight 70,000) 1.0 g/m² was applied in advance, basis weight 12 g/cm², thickness 40 µm], such that the thickness after drying became 60 µm. The substrate was dried at 100°C for 5 minutes and adhered to a release liner (same as that used in Example 1) to give an adhesive preparation of the present invention.

### Example 4 preparation of adhesive preparation

An adhesive solution containing 35 parts by weight (solid content) of a copolymer comprising 2-ethylhexyl acrylate (95 parts by weight), and acrylic acid (5 parts by weight), isopropyl myristate (40 parts by weight) and methoxamine base (25 parts by weight) as a drug in toluene/methanol=1/1 mixture (300 parts by weight) was prepared. This adhesive solution was applied to the surface of a nonwoven fabric of a substrate comprising a synthetic resin film (polyester film, thickness 6 µm) and a polyester nonwoven fabric [polyethyleneimine (EPOMIN SP-006, number average molecular weight 6,000) 1.5 g/m² was applied in advance, basis weight 12 g/cm², thickness 40 µm], such that the thickness after drying became 80 µm. The substrate was dried at 100°C for 5 minutes and adhered to a release liner (same as that used in Example 1) to give an adhesive preparation of the present invention.

### Comparative Examples 1-4 preparation of adhesive sheet and adhesive preparation

In the same manner as in Examples 1-4 except the use of polyethyleneimine, adhesive sheets and adhesive preparations were respectively obtained.

### Comparative Example 5 preparation of adhesive sheet

An adhesive solution containing 60 parts by weight (solid content) of a copolymer comprising 2-ethylhexyl acrylate (95 parts by weight) and acrylic acid (5 parts by weight), and isopropyl myristate (40 parts by weight in toluene/methanol=1/1 mixture (300 parts by weight) was prepared. This adhesive solution was applied to the surface of a nonwoven fabric of a substrate comprising a synthetic resin film (polyester film, thickness 6 µm) and a polyester nonwoven fabric [polyethyleneimine (EPOMIN P-1000, number average molecular weight 70,000) 0.003 g/m² was applied in advance, basis weight 8 g/cm², thickness 30 µm], such that the thickness after drying became 40 µm. The substrate was dried at 100°C for 5 minutes and adhered to a release liner (same as that used in Example 1) to give an adhesive sheet.

### Comparative Example 6 preparation of adhesive sheet

In the same manner as in Comparative Example 5 except that a substrate wherein a polyester film (thickness 6 µm) and a polyester nonwoven fabric [polyethyleneimine (EPOMIN P-1000, number average molecular weight 70,000) 20 g/m² was applied in advance, basis weight 8 g/cm², thickness 30 µm] were adhered to each other, an adhesive sheet was prepared.

The adhesive sheets and adhesive preparations obtained in Examples and Comparative Examples were respectively preserved at 60°C for 48 hours and subjected to the following Test Examples 1-3.

### Test Example 1 holding power test

The adhesive sheets and adhesive preparations obtained in Examples and Comparative Examples were cut out in 1×5 cm to give samples and the opposite edge (1×2 cm) of the samples was adhered to a bakelite board (2.5×4 cm). A roller was reciprocated once at a load of 850 g in the longitudinal direction on the part of the sample where it was adhered to the bakelite board. The bakelite board with the sample adhered thereto was stood in a thermostat heated to 40°C for 30 minutes. A load of 150 g was hung on the edge opposite from the edge of the sample adhered to the bakelite board, and the time necessary for the sample to fall down was measured. The measured time is shown in the following Table 1 as the holding power of the adhesive.

### Test Example 2 adhesive residue evaluation test after adhesion to rabbit skin

The adhesive sheets and adhesive preparations obtained in Examples and Comparative Examples were cut out in an area of 10 cm² to give samples. They were adhered to the skin of the back of New Zealand white rabbits, which had been sheared and shaved, for 24 hours and peeled off. The adhesive residue on each rabbit was evaluated as follows according to the ratio of the area of the adhesive residue to the area of the skin surface. The results are shown in the following Table 1.
- ○:: area of adhesive residue 0 to less than 3% (almost no adhesive residue is observed)
- Δ:: area of adhesive residue 3 to less than 15% (adhesive residue is somewhat found on the edge of the adhesive)
- ×:: area of adhesive residue 15 to 100% (adhesive residue is found on the entirety of the adhesive residue)

### Test Example 3 evaluation test of ease of taking out after preservation

The adhesive sheets and adhesive preparations obtained in Examples and Comparative Examples were cut out in an area of 10 cm² to give samples. They were each sealed in a package (made of polyacrylonitrile) and preserved at 40°C for 3 months. After preservation, the ease of taking out the adhesive sheet and the adhesive preparation from the package was evaluated according to the following evaluation criteria. The results are shown in the following Table 1.
- ○:: easily taken out without protrusion of adhesive
- Δ:: adhesive protrudes somewhat, which prevents easy taking out
- ×:: much adhesive protrudes and the product cannot be taken out

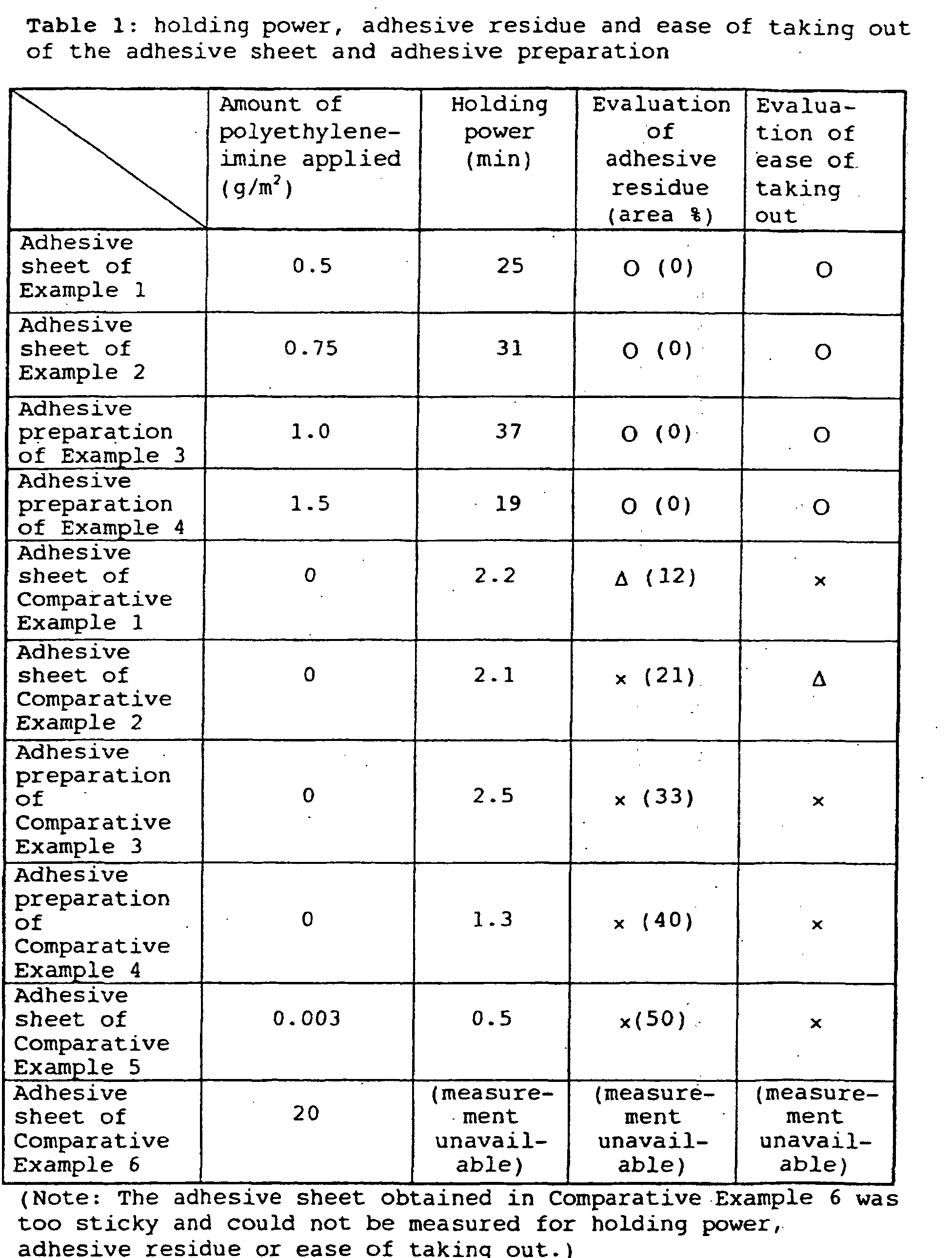

From the above-mentioned test results, it is clear that the adhesive sheet and the adhesive preparation of the present invention have superior characteristics of high holding power, less adhesive residue and easy taking out from a package.

As mentioned above, the adhesive sheet and the adhesive preparation of the present invention can have optimally adjusted cohesiveness of an adhesive layer by applying polyethyleneimine to at least the surface of a nonwoven fabric. This has an effect that the adhesive sheet of the present invention is free of adhesive residue on the skin surface upon peeling after adhesion to the skin. In addition, the adhesive sheet and the adhesive preparation of the present invention can be easily taken out from a package after preservation in the package. Because the adhesive sheet and the adhesive preparation of the present invention contain polyethyleneimine at least on the surface of the nonwoven fabric, rather than in an adhesive layer, the adhesive solution can have a longer pot life. Even when a drug inhibiting crosslinking of the base drug and the like is added to an adhesive layer, the cohesiveness of the adhesive layer can be advantageously increased.

## Claims

1. An adhesive sheet comprising a substrate comprising a synthetic resin film and a nonwoven fabric laminated on one side of the film, and an adhesive layer formed on the nonwoven fabric, wherein the nonwoven fabric comprises polyethyleneimine at least on a surface of the nonwoven fabric.

2. The adhesive sheet according to claim 1, wherein the polyethyleneimine is contained in a proportion of 0.01-10 g/m² and at least on the surface of the nonwoven fabric.

3. The adhesive sheet according to claim 1 or 2, wherein the polyethyleneimine has a number average molecular weight of 300-100,000.

4. The adhesive sheet according to claim 1, wherein the adhesive layer comprises an adhesive containing a carboxyl group.

5. The adhesive sheet according to claim 4, wherein the adhesive sheet comprises a polymer containing the following (a) and (b):
(a) at least one C₄₋₁₃ alkyl (meth)acrylate monomer unit; and
(b) at least one ethylenically unsaturated monomer unit containing a carboxyl group.

6. The adhesive sheet according to any of claims 1 to 5, wherein the synthetic resin film is made from a polyester and the nonwoven fabric is made from a polyester.

7. An adhesive preparation comprising a drug in an adhesive layer of the adhesive sheet according to any of claims 1 to 6.

8. A production method of the adhesive sheet of claim 1, which comprises forming an adhesive layer on a nonwoven fabric containing polyethyleneimine.

9. A production method of the adhesive preparation of claim 7, which comprises forming an adhesive layer containing a drug on a nonwoven fabric containing polyethyleneimine.

## Patentansprüche

1. Heftpflaster, das ein Substrat, umfassend eine Folie eines synthetischen Harzes und ein Faservlies, das an eine Seite der Folie laminiert ist, und eine Klebstoffschicht umfasst, die auf dem Faservlies ausgebildet ist, wobei das Faservlies wenigstens auf einer Oberfläche des Faservlieses Polyethylenimin umfasst.

2. Heftpflaster gemäß Anspruch 1, in dem das Polyethylenimin in einem Verhältnis von 0,01 - 10 g/m² und wenigstens auf der Oberfläche des Faservlieses enthalten ist.

3. Heftpflaster gemäß den Ansprüchen 1 oder 2, wobei das Polyethylenimin ein Zahlenmittel der Molmasse von 300 bis 100 000 hat.

4. Heftpflaster gemäß Anspruch 1, wobei die Klebstoffschicht einen Klebstoff umfasst, der eine Carboxylgruppe enthält.

5. Heftpflaster gemäß Anspruch 4, wobei das Heftpflaster ein Polymer umfasst, das die folgendenden (a) und (b):
(a) wenigstens eine C₄₋₁₃-Alkyl(meth)acrylat-Monomereinheit und
(b) wenigstens eine ethylenisch ungesättigte Monomereinheit, die eine Carboxylgruppe enthält,
enthält.

6. Heftpflaster gemäß irgendeinem der Ansprüche 1 bis 5, in dem die Folie aus dem synthetischen Harz aus einem Polyester hergestellt wird, und das Faservlies aus einem Polyester hergestellt wird.

7. Klebendes Präparat, umfassend einen Wirkstoff in einer Klebstoffschicht des Heftpflasters gemäß irgendeinem der Ansprüche 1 bis 6.

8. Herstellungsverfahren des Heftpflasters gemäß Anspruch 1, das die Bildung einer Klebstoffschicht auf einem Polyethylenimin-enthaltenden Faservlies umfasst.

9. Herstellungsverfahren des Heftpflasters gemäß Anspruch 7, das die Bildung einer Klebstoffschicht, die einen Wirkstoff enthält, auf einem Polyethylenimin-enthaltenden Faservlies umfasst.

## Revendications

1. Feuille adhésive comprenant un substrat comprenant un film de résine synthétique et un tissu non tissé laminé sur un côté du film, et une couche adhésive formée sur le lissu non tissé, dans laquelle le tissu non tissé comprend une polyéthylèneimine au moins sur une surface du tissu non tissé.

2. Feuille adhésive selon la revendication 1, dans laquelle la polyéthylèneimine est contenue en une proportion comprise dans la plage allant de 0,01 à 10 g/m² et au moins sur la surface du tissu non tissé.

3. Feuille adhésive selon la revendication 1 ou 2, dans laquelle la polyéthylèneimine a un poids moléculaire moyen compris dans la plage allant de 300 à 100000.

4. Feuille adhésive selon la revendication 1, dans laquelle la couche adhésive comprend un adhésif contenant un groupe carboxyle.

5. Feuille adhésive selon la revendication 4, dans laquelle la feuille adhésive comprend un polymère contenant (a) et (b) suivants :
(a) au moins une unité de monomère de (méth)acrylate d'alkyle en C₄-C₁₃ ; et
(b) au moins une unité de monomère insaturé de manière éthylénique contenant un groupe carboxyle.

6. Feuille adhésive selon l'une quelconque des revendicatione 1 à 5, dans laquelle le film de résine synthétique est préparé à partir d'un polyester et le tissu non tissé est préparé à partir d'un polyester.

7. Préparation adhésive comprenant un médicament dans une couche adhésive de la feuille adhésive selon l'une quelconque des revendications 1 à 6.

8. Procédé de production de la feuille adhésive selon la revendication 1, qui comprend la formation d'une couche adhésive sur un tissu non tissé contenant une polyéthylèneimine.

9. Procédé de production de la préparation adhésive selon la revendication 7, qui comprend la formation d'une couche adhésive contenant un médicament sur un tissu non tissé contenant une polyéthylèneimine.
